# FASCICULE DE BREVET EUROPEEN

(11) **EP 0 582 511 B1**
(45) Date de publication et mention de la délivrance du brevet: **11.06.1997**
(21) Numéro de dépôt: 93401979.5
(22) Date de dépôt: 30.07.1993
(51) Int. Cl.: A61M 5/32, A61M 5/46

(54) **Dispositif de protection d'aiguille d'injection**
Injektionsnadel-Schutzvorrichtung
Protection device for injection needle

(30) Priorité: 31.07.1992 FR 9209516
(43) Date de publication de la demande: 09.02.1994
(73) Titulaire: MATEF, F-92500 Rueil Malmaison (FR); Trapp, Claude, 92500 Rueil Malmaison (FR)
(72) Inventeur: Trapp, Claude, F-92500 Rueil Malmaison (FR)
(74) Mandataire: Hud, Robert

(56) Documents cités:
- EP-A- 0 485 345
- WO-A-92/04073
- FR-A- 978 344
- US-A- 4 897 083
- US-A- 4 961 730
- US-A- 5 106 380

## Description

L'invention s'applique à des appareils d'injection, à usage principalement médical, mais aussi vétérinaire ou alimentaire.

De tels appareils sont connus dans le domaine des instruments médicaux. Ainsi le brevet des Etats-Unis d'Amérique N° 3 880 163 montre une pièce à main contenant une seringue à piston équipée d'une aiguille d'injection, et deux ressorts, l'un pour pousser une catapulte portant la seringue pour introduire mécaniquement l'aiguille dans la chair du patient à traiter, l'autre porté par la catapulte pour pousser le piston dans la seringue et injecter le liquide de traitement à travers l'aiguille.

Celle-ci est protégée avant emploi par un capuchon amovible et entourée en outre par une garde tubulaire démontable et réglable, emmanchée sur l'extrémité de la pièce à main et pouvant toucher la peau du patient.

L'utilisation de ces divers éléments, et en particulier leur mise en place et leur retrait de la pièce à main, nécessite de nombreux mouvements et une grande attention de la part du praticien pour éviter de se piquer lui-même.

En outre il est récemment apparu nécessaire que tous les éléments ayant pu toucher le patient soient jetables pour éviter de contaminer une autre personne.

L'invention satisfait à cette nécessité, et en outre ne demande au praticien que des manipulations simples et sûres des divers éléments. Elle s'applique de préférence, mais non nécessairement, à l'appareil médical comportant une pièce à main avec catapulte d'injection décrit dans la demande de brevet en France N° 92.03870 déposée le 31 mars 1992 et publiée sous le numero FR-A-2689017 apres la date de priorité de la demande.

Par le document EP-A-0 485 345, on connait une seringue à usage unique, qui comporte une garde de protection de l'aiguille. Cette garde est emmanchée sur la seringue et peut coulisser entre une position de sécurité dans laquelle des surfaces de connexion qu'elle porte engagent des surfaces de connexion complémentaires portées par l'embout de support de l'aiguille, et une position d'utilisation où ses surfaces de connexion engagent d'autres surfaces de connexion portées par la seringue.

Par le document FR-A-978 344, on connait un appareil actionnant, par la détente d'un ressort, une aiguille à injection. Cet appareil comporte, autour de l'aiguille, une garde fixe qui présente une échancrure oblique.

Le document WO-A-9204073, qui correspond au préambule de la revendication 1, décrit un dispositif de protection d'une aiguille d'injection dont un embout est engagé dans une extrémité d'une conduite de liaison. Ce dispositif comporte une garde qui entoure l'aiguille et qui peut être emmanchée sur une pièce de verrouillage fixée sur l'extrémité de la conduite de liaison pour dégager l'aiguille. L'embout et la garde présentent des surfaces de connexion mutuelle qui sont déconnectées l'une de l'autre lors de l'emmmanchement de la garde sur la pièce de déverrouillage, et ces surfaces de connexion présentent des portions coopérantes inclinées qui assurent l'entraînement de l'aiguille lors de l'extraction de la garde de son emmanchement avec la pièce de déverrouillage.

Selon la présente invention, un appareil d'injection avec dispositif de protection de l'aiguille d'injection, comprend une aiguille d'injection pourvue d'un embout, une garde entourant l'aiguille et coulissant par rapport à celle-ci entre une position de dégagement dans laquelle la garde s'emmanche dans une pièce de retenue et une position de protection dans laquelle coopèrent des parties de connexion mutuelle de l'embout et de la garde, et une pièce de poussée interne à la garde et pouvant appuyer sur l'embout. Il se caractérise en ce que la dite pièce de retenue est fixée sur le guide-support de l'appareil d'injection et entoure une catapulte qui coulisse dans le dit guide-support, et à laquelle se fixe une partie cylindrique de l'embout sous l'effet de la pièce de poussée, la dite pièce de poussée étant distincte de la garde et présentant la forme d'un capuchon qui est inséré dans la garde de façon que l'extrémité fermée reste exposée à une poussée de fixation de l'embout à la catapulte.

Pour bien faire comprendre le dispositif selon l'invention on en décrira ci-après, à titre d'exemples sans caractère limitatif, plusieurs formes d'exécution en référence au dessin schématique annexé dans lequel :
la figure 1a est une vue en coupe diamétrale partielle de l'extrémité d'un appareil d'injection;
la figure 1b est, disposée en vis à vis de la figure 1a, une vue en coupe diamétrale partielle d'une première forme d'exécution d'un dispositif de protection selon l'invention;
la figure 2 est une vue en coupe diamétrale partielle montrant la fixation du dispositif de la figure 1b sur l'extrémité de la catapulte de l'appareil d'injection;
la figure 3 est une vue correspondant à la figure 2, qui montre les mêmes pièces en position d'injection;
la figure 4 est une vue en coupe diamétrale d'une variante du capuchon de poussée;
la figure 5 est une vue en coupe diamétrale d'une autre variante d'un capuchon monté avec un dispositif de protection selon l'invention;
la figure 6 est une vue en coupe diamétrale partielle d'une autre forme d'exécution du dispositif selon l'invention;
la figure 7 est une vue correspondant à la figure 9, mais avec les pièces en position d'injection;
la figure 8 est une vue correspondant aux figures 6 et 7, montrant les mêmes pièces bloquées entre elles et sans le capuchon.

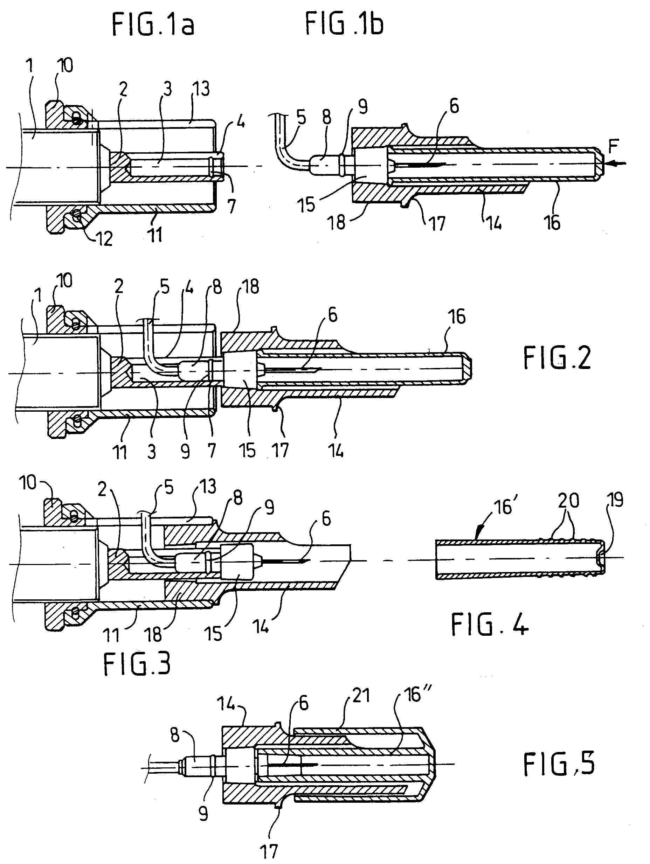

L'appareil d'injection comporte, pour toutes les formes d'exécution décrites et comme indiqué à la figure 1a, un guide-support cylindrique 1, à l'intérieur duquel peut coulisser une catapulte dont l'extrémité 2 comporte un alésage 3 pourvu d'une fente radiale 4 susceptible de laisser passer le tube souple 5 d'alimentation de l'aiguille d'injection 6 qui sont représentés sur la figure 1b.

En outre une rainure circulaire 7 de cet alésage permet, par élasticité de l'alésage 3, de maintenir l'embout de fixation qui comporte une partie cylindrique 8 de diamètre adapté à l'alésage 3 et un bourrelet circulaire 9 pouvant pénétrer dans la rainure 7.

L'appareil d'injection, pour sa partie non représentée au dessin, est par exemple du type décrit dans la demande de brevet français N° 92.03870 précitée.

Autour du guide 1, fileté extérieurement, se trouve un écrou 10 qui retient longitudinalement une pièce d'emmanchement 11 au moyen d'un anneau brisé élastique 12. La pièce d'emmanchement 11 présente une fente radiale 13 susceptible de laisser passer le tube souple 5 et qu'on peut orienter dans le plan de la fente 4 en tournant la pièce 11 autour de l'anneau 12. Par rotation de l'écrou 10 on peut régler la position longitudinale de la pièce 11, ce qui règle la profondeur de pénétration de l'aiguille 6 comme on le verra plus loin.

Selon la première forme d'exécution représentée à la figure 1b, le dispositif selon l'invention comprend une garde 14 en connexion avec l'embout de fixation de l'aiguille 6, cette connexion résultant de l'introduction d'un cône mâle 15 de l'embout dans un cône femelle de la garde 14.

La garde 14 est constituée par un tube dont l'alésage entoure coaxialement l'aiguille 6, et elle est échancrée obliquement en face de l'extrémite de celle-ci. Cette échancrure permet au praticien de viser le point d'injection au centre de l'arc de cercle de l'extrémité mise en contact avec la peau du patient.

L'alésage de la garde 14 reçoit à frottement doux un capuchon 16, amovible, qui protège l'aiguille 6. L'extrémité ouverte de ce capuchon prend appui sur la face de grand diamètre du cône 15.

En exerçant une poussée sur le capuchon, selon la flèche F de la figure 1b, et après avoir fait passer le tube 5 dans les fentes 4 et 13, on peut pousser le cylindre 8 de l'embout dans l'alésage 3 jusqu'à ce que le bourrelet 9 se clipse dans la rainure 7. La figure 2 montre la position des divers éléments à cet instant, où l'aiguille 6 est fixée à la catapulte.

La fixation peut aussi être réalisée par poussée radiale du cylindre 8 sur l'extrémité 2, à travers la fente 4. Dans ce cas, le capuchon 16 ne sert pas de pièce de poussée.

Ensuite, pour libérer l'aiguille de sa garde 14, on pousse alors celle-ci par sa collerette 17 pour introduire, comme on le voit à la figure 3, la partie cylindrique 18 de la garde 14 dans la pièce d'emmanchement 11, cet emmanchement se faisant à frottement doux. L'extrémité de l'aiguille 6 est proche alors de l'extrémité échancrée de la garde 14 et ce d'autant plus que l'écrou 10 est plus engagé, par vissage, sur le guide 1. Simultanément à cet emmanchement, le cône 15 sort du cône femelle de la garde 14, en désolidarisant celle-ci d'avec l'aiguille 6.

L'enlèvement du capuchon 16, comme on le voit à la figure 3, permet alors de procéder à l'injection, ou à des injections successives, dont la profondeur est déterminée par le mouvement de la catapulte 2 et par un appui léger de la garde 14 autour du point d'injection.

Après l'acte d'injection on peut, ou non, remettre le capuchon 16. On tire ensuite la garde 14 pour l'extraire de la pièce d'emmanchement 11 et la solidariser de nouveau à l'aiguille par le cône 15 de son embout revenant à la position de la figure 2. Une traction plus ferme utilisant en particulier la collerette 17 arrache alors l'embout d'avec la catapulte 2, ce qui accentue fortement l'engagement du cône 15 dans la garde et évite ainsi toute séparation qui mettrait l'aiguille 6 à nu. On peut alors en toute sécurité démonter le tube souple 5 et jeter l'ensemble d'injection, éventuellement infecté par l'acte d'injection.

Le capuchon 16' représenté en variante à la figure 4, spécialement adapté à l'usage de pièce de poussée selon l'invention, comporte une dépression légère 19 à son extrémité fermée, pour éviter un dérapage du doigt du praticien lors de l'action de poussée, ainsi que des stries latérales 20 ayant le même objet. Ces détails rappellent en outre au praticien l'utilisation spécifique de ce capuchon en tant que pièce de poussée pour fixer l'aiguille 6 sur l'appareil d'injection.

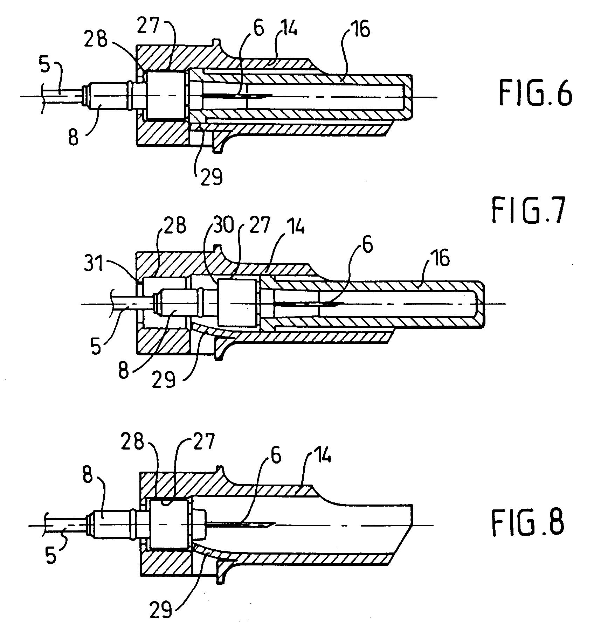

Le capuchon 16'' représenté comme autre variante à la figure 5 comporte en outre une gaine 21 qui entoure au moins partiellement la garde 14 ce qui oblige pratiquement le praticien à l'utiliser en premier lieu en tant que pièce de poussée, sans agir sur la garde 14 avant que la fixation soit réalisée sur la catapulte.

Aux figures 6, 7 et 8, on a représenté une autre forme d'exécution de l'invention.

Dans ce cas les surfaces de connexion 27,28 sont cylindriques, ce qui est plus facile à réaliser que des surfaces coniques. Les tolérances de fabrication sont en outre larges car les dites surfaces n'ont pas à assurer ici un serrage pour obtenir une retenue de l'embout dans la garde 14. En effet cette retenue est alors réalisée au moyen d'un cliquet 29, articulé à la périphérie de la garde 14 avec effet élastique pour qu'il tende à pénétrer à l'intérieur de l'alésage de la garde.

Initialement le capuchon 16 maintient le cliquet 29 écarté et, dans son mouvement de poussée, puis d'emmanchement de la garde 14, le cliquet passe autour de la surface de connexion 27 de l'embout, comme le montre la figure 7. En fin d'opération, après retrait du capuchon 16, la traction de la garde connecte le cylindre 27 avec le cylindre 28, jusqu'à ce qu'un épaulement 30 de l'embout bute contre un épaulement 31 de la garde 14 ce qui permet de libérer l'embout d'avec la catapulte.

Simultanément le cliquet 29 se rapproche de l'axe de l'aiguille et empêche celle-ci de glisser dans la garde, en butant sur l'embout de l'aiguille 6, comme le montre la figure 8.

Une variante d'emploi de cette autre forme d'exécution consiste à disposer préalablement en retrait le capuchon 16 pour laisser libre le cliquet 29. On peut de ce fait fixer l'embout 8 à la catapulte 2 en poussant la garde 14 dont le cliquet 29 s'appuie sur l'embout de l'aiguille 6, en constituant ainsi la pièce de poussée selon l'invention.

Ensuite une légère pression sur le capuchon 16 écarte le cliquet 29, et une nouvelle poussée sur la garde 14 permet de l'emmancher dans la pièce 11 de la figure 1a.

En fin d'opération le cliquet 29 joue le même rôle de retenue que précédemment.

On comprendra que la description ci-dessus a été donnée à simple titre d'exemple, sans caractère limitatif, et que des modifications ou des adjonctions constructives pourraient lui être apportées sans sortir du cadre de l'invention comme defini par les revendications suivantes.

## Revendications

1. Appareil d'injection avec dispositif de protection de l'aiguille d'injection, comprenant une aiguille d'injection (6) pourvue d'un embout (15), une garde (14) entourant l'aiguille (6) et coulissant par rapport à celle-ci entre une position de dégagement dans laquelle la garde (14) s'emmanche dans une pièce de retenue (11) et une position de protection dans laquelle coopèrent des parties de connexion mutuelle (15,27,30 et 28,31) de l'embout (15) et de la garde (14), et une pièce de poussée (16) interne à la garde (14) et pouvant apppuyer sur l'embout (15), caractérisé en ce que la dite pièce de retenue (11) est fixée sur le guide-support (1) de l'appareil d'injection et entoure une catapulte (2) qui coulisse dans le dit guide-support (1) et à laquelle se fixe une partie cylindrique (8) de l'embout (15) sous l'effet de la pièce de poussée (16), la dite pièce de poussée (16) étant distincte de la garde (14) et présentant la forme d'un capuchon qui est inséré dans la garde (14) de façon que l'extrémité fermée reste exposée à une poussée de fixation de l'embout (15) à la catapulte (2).

2. Dispositif selon la revendication 1, caractérisé en ce que la connexion comporte une retenue assurée par un cliquet (29) articulé sur la garde (14).

## Claims

1. Injection apparatus with a protection device for the injection needle, comprising an injection needle (6) provided with a connector (15), a guard (14) surrounding the needle (6) and slidable in relation thereto between a disengaged position in which the guard (14) is fitted into a retaining part (11) and a protection position in which the mutual connection parts (15,27,30 and 28,31) of the connector (15) and of the guard (14) cooperate, and a thrust part (16) which is inside the guard (14) and may bear on the connector (15), characterised in that said retaining part (11) is secured on the guide support (1) of the injection device and surrounds a catapult (2) which slides in said guide-support (1) and to which a cylindrical part (8) of the connector (15) is secured under the action of the thrust part (16), said thrust part (16) being separate from the guard (14) and having the shape of a cap which is inserted into the guard (14) in such a manner that the closed extremity remains exposed to a thrust for securing the connector (15) to the catapult (2).

2. Device according to Claims 1,
characterised in that the connection comprises a retention ensured by means of a ratchet (29) articulated on the guard (14).

## Patentansprüche

1. Injektionsgerät mit einer Schutzvorrichtung für die Injektionsnadel, das folgendes umfaßt: eine mit einem Endstück (15) versehene Injektionsnadel (6), eine die Nadel (6) umgebende Sicherheitsvorrichtung (14), die bezüglich ersterer zwischen einer Ausrückpösition, in der die Sicherheitsvorrichtung (14) in einem Halteteil (11) eingesetzt ist, und einer Schutzposition, in der die Teile (15, 27, 30 und 28, 31) zum Miteinanderverbinden des Endstücks (15) und der Sicherheitsvorrichtung (14) zusammenwirken, verschiebbar ist, und ein sich in der Sicherheitsvorrichtung (14) befindendes Schiebeteil (16), das sich an dem Endstück (15) stützen kann, dadurch gekennzeichnet, daß das Halteteil (11) an der Stützführung (1) des Injektionsgeräts befestigt ist und eine Ausstoßvorrichtung (2) umgibt, die in der Stützführung (1) verschiebbar ist und an der ein zylindrischer Teil (8) des Endstücks (15) unter der Wirkung des Schiebeteils (16) befestigt ist, wobei das Schiebeteil (16) von der Sicherheitsvorrichtung (14) getrennt ist und die Form einer Kappe aufweist, die so in der Sicherheitsvorrichtung (14) eingesetzt ist, daß das geschlossene Ende einer Befestigungslängskraft des Endstücks (15) auf die Ausstoßvorrichtung (2) ausgesetzt ist.

2. Vorrichtung nach Anspruch 1, dadurch gekennzeichnet, daß die Verbindung eine Halterung in Form einer an der Sicherheitsvorrichtung (14) angelenkten Sperrklinke (29) umfaßt.
